# EUROPÄISCHE PATENTSCHRIFT

(11) **EP 0 741 681 B1**
(45) Veröffentlichungstag und Bekanntmachung des Hinweises auf die Patenterteilung: **20.05.1998**
(21) Anmeldenummer: 95906983.2
(22) Anmeldetag: 20.01.1995
(51) Int. Cl.: C07B 37/04, C07C 41/30, C07C 43/215, C07D 307/33, C07D 309/12, C07F 7/18

(54) **VERFAHREN ZUR HERSTELLUNG VON 3-ARYL-PROPINEN UND NEUE 3-ARYL-PROPINE**
PROCESS FOR PRODUCING 3-ARYL PROPINES AND NOVEL 3-ARYL PROPINES
PROCEDE DE PRODUCTION DE 3-ARYL-PROPYNES ET DE NOUVEAUX 3-ARYL-PROPYNES

(30) Priorität: 01.02.1994 DE 4402915
(43) Veröffentlichungstag der Anmeldung: 13.11.1996
(73) Patentinhaber: BAYER AG, 51368 Leverkusen (DE)
(72) Erfinder: LAUE, Christian, D-40789 Monheim (DE)
(86) Internationale Anmeldenummer: EP9500208
(87) Internationale Veröffentlichungsnummer: WO9521144

(56) Entgegenhaltungen:
- GAZZETTA CHIMICA ITALIANA, Bd. 120, 1990 Seiten 783-791, R. ROSSI ET AL 'A novel and efficient method to prepare 3-(hetero)aryl-1-propynes and its application to the stereoselective synthesis of (2E,4E)-N-(2-methylpropyl)-6-(2-thienyl)-2 ,4-hexadienamide, piperovatine and (E)-N-(2-methylpropyl)-6-(4-methoxyphenyl) -4-hexyne-2-enamide'
- JOURNAL OF ORGANIC CHEMISTRY, Bd. 17,Nr. 3, März 1952 EASTON US, Seiten 475-481, T. L. JACOBS ET AL 'The rearrangement of aryl-substituted propynes to allenes'
- JOURNAL OF ORGANOMETALLIC CHEMISTRY, Bd. 93,Nr. 2, 1975 LAUSANNE CH, Seiten 259-263, H. A. DIECK ET AL 'Palladium catalyzed synthesis of aryl, heterocyclic and vinylic acetylene derivatives'

## Beschreibung

Die Erfindung betrifft ein Verfahren zur Herstellung von 3-Aryl-propinen durch Palladium-katalysierte Benzylierung von Alkinen und einige neue 3-Aryl-propine.

Bekannt ist die Herstellung von 3-Aryl-propinen durch Benzylierung von Acetylenen unter Metallierung durch die stark basischen und nucleophilen Grigard-Reagenzien [Jakobs, Stinger, J. Org. Chem. 17, 475 (1952)].

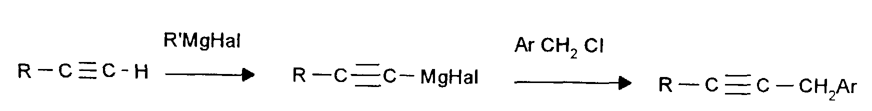

Weiterhin bekannt (E. Rossi et al. Gazz. Chim. Ital. 120, 783 (1990) ist die Herstellung von 3-(4-Methoxy-phenyl)-propinen durch Metallierung von Acetylenen mit der starken, nucleophilen Base Ethylmagnesiumbromid und Umsetzung mit einem Benzylchlorid unter Cu(I)-Katalyse. Dieses Verfahren hat den Nachteil, das Cu(I)-Acetylen-Verbindungen als explosiv betrachtet werden müssen.

Beide bisher bekannten Verfahren haben den Nachteil, daß wegen der verwendeten stark nucleophilen Basen Kupplungskomponenten mit elektrophilen Gruppen, wie z.B. Carbonyl-Gruppen, nicht umgesetzt werden können, weil diese alkyliert und/oder an der α-Position deprotoniert würden.

Es wurde nun ein Verfahren zur Herstellung von 3-Aryl-propinen der allgemeinen Formel (I) 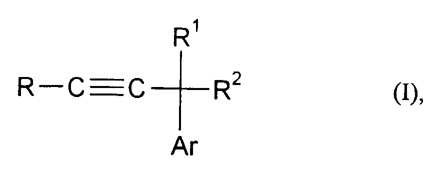 in welcher
- Ar: - für Aryl mit 6 bis 10 Kohlenstoffatomen steht, das gegebenenfalls durch Alkyl, Alkylsulfonyl oder Alkoxycarbonyl mit bis zu 10 Kohlenstoffatomen oder durch Alkoxy mit bis zu 8 Kohlenstoffatomen substituiert sein kann, wobei die Alkoxygruppen wiederum durch Phenoxy oder Alkoxy mit bis zu 6 Kohlenstoffatomen untersubstituiert sein können oder
- für einen 5- bis 6-gliedrigen, gegebenenfalls Benzo-kondensierten heterocyclischen Ring steht, der als Heteroatome bis zu 2 Stickstoff-, Sauerstoff- und/oder Schwefelatome enthalten kann und der gegebenenfalls durch Alkyl, Alkoxy oder Alkoxycarbonyl mit bis zu 6 Kohlenstoffatomen oder durch Phenyl substituiert sein kann,
- R: - für Wasserstoff oder Alkyl mit bis zu 10 Kohlenstoffatomen steht, das gegebenenfalls durch eine Gruppe -OR³, -NHR³ oder -NR³₂ und/oder Alkoxycarbonyl mit bis zu 6 Kohlenstoffatomen substituiert sein kann oder
- für Aryl mit 6 bis 10 Kohlenstoffatomen steht, das gegebenenfalls durch Alkyl, Alkoxy oder Alkoxycarbonyl mit bis zu 8 Kohlenstoffatomen substituiert ist oder
- für eine Trialkylsilyl- oder eine Alkyldiphenyl silyl-Gruppe steht, wobei die Alkylgruppen bis zu 6 Kohlenstoffatomen enthalten können oder
- für eine Gruppe der Formeln 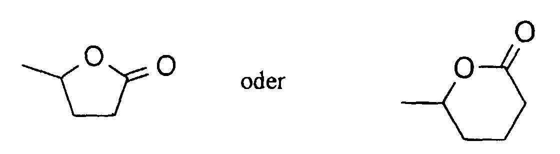 steht,
- R¹, R²: gleich oder verschieden sind und
- für Wasserstoff, Alkyl mit bis zu 6 Kohlenstoffatomen oder Aryl mit 6 bis 10 Kohlenstoffatomen stehen oder
- gemeinsam mit dem dazwischen liegenden Kohlenstoffatom einen Cyclopentyl- oder Cyclohexylring bilden
und
- R³: für Wasserstoff, Alkyl mit bis zu 6 Kohlenstoffatomen, Acyl mit bis zu 6 Kohlenstoffatomen, Benzoyl, Tetrahydropyranyl, Trialkylsilyl oder Alkyldiphenylsilyl steht, wobei die Alkylgruppen hier jeweils bis zu 6 Kohlenstoffatomen enthalten
gefunden, das dadurch gekennzeichnet ist, daß man
Benzylverbindungen der allgemeinen Formel (II) 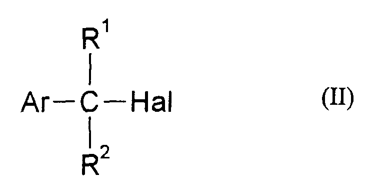 in welcher
- Hal: für Chlor oder Brom steht und
- Ar, R¹ und R²: die bei Formel (I) angegebene Bedeutung haben,
mit Alkinen der allgemeinen Formel (III)

H―C≡C―R (III),

in der R die bei Formel (I) angegebene Bedeutung hat,
in Gegenwart einer Palladium-Verbindung gegebenenfalls in Anwesenheit von Phosphinen, mit Hilfe von schwachen Basen in inerten Lösungsmitteln umsetzt.

Das erfindungsgemäße Verfahren zur Herstellung von 3-Aryl-propinen hat den Vorteil, daß als Basen keine metallorganischen Reagenzien wie Buthyllithium oder Grignard-Reagentien verwendet werden, sondern mildere Basen wie Amine oder Salze schwacher Basen (z.B. NaHCO₃). Diese ersparen aufwendige Maßnahmen zum Feuchtigkeitsausschluß. Insbesondere ermöglicht das erfindungsgemäße Verfahren die Herstellung von Arylpropinen der allgemeinen Formel (I) mit elektrophilen Substituenten, wie Carbonylgruppen, die mit den üblicherweise verwendeten starken, nucleophilen Basen alkyliert oder deprotoniert würden.

Bevorzugt werden nach dem erfindungsgemäßen Verfahren solche 3-Aryl-propine der allgemeinen Formel (I) hergestellt
in welcher
- Ar: - für Phenyl oder Naphthyl steht, das gegebenenfalls durch Alkyl oder Alkoxycarbonyl mit bis zu 8 Kohlenstoffatomen oder durch Alkoxy mit bis zu 6 Kohlenstoffatomen substituiert sein kann, wobei die Alkoxygruppen wiederum durch Phenoxy oder Alkoxy mit bis zu 4 Kohlenstoffatomen substituiert sein können oder
- für Thienyl, Furyl, Pyridyl, Pyrimidyl, Pyrazinyl, Chinolyl, Isochinolyl, Benzofuryl, Imidazolyl, Benzimidazolyl oder Indolyl steht, die gegebenenfalls durch Alkyl oder Alkoxy mit bis zu 4 Kohlenstoffatomen oder durch Phenyl substituiert sind,
- R: - für Wasserstoff oder Alkyl mit bis zu 8 Kohlenstoffatomen steht, das gegebenenfalls durch eine Gruppe -OR³ und/oder Alkoxycarbonyl mit bis zu 4 Kohlenstoffatomen substituiert ist oder
- für Phenyl oder Naphthyl steht, das gegebenenfalls durch Alkyl oder Alkoxy mit bis zu 6 Kohlenstoffatomen substituiert ist oder
- für eine Trialkylsilyl- oder Alkyldiphenylsilylgruppe steht, wobei die Alkylgruppen bis zu 4 Kohlenstoffatomen enthalten können oder
- für eine Gruppe der Formeln 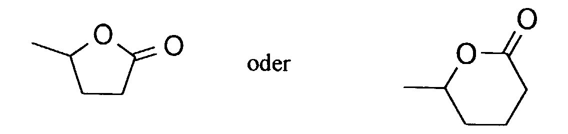 steht,
- R¹, R²: gleich oder verschieden sind und
- für Wasserstoff, Alkyl mit bis zu 4 Kohlenstoffatomen oder für Phenyl stehen und
- R³: für Wasserstoff, Alkyl mit bis zu 4 Kohlenstoffatomen, Acetyl oder Benzoyl steht oder für Tetrahydropyranyl, Trialkylsilyl oder Alkyldiphenylsilyl steht, wobei die Alkylgruppen jeweils bis zu 4 Kohlenstoffatome enthalten.

Besonders bevorzugt werden solche 3-Aryl-propine der allgemeinen Formel (I) hergestellt, in welchen
- Ar: für Phenyl steht, das gegebenenfalls durch Alkyl mit bis zu 4 Kohlenstoffatomen oder Alkoxy mit bis zu 6 Kohlenstoffatomen substituiert ist, wobei die Alkoxygruppe wiederum durch Phenoxy oder Alkoxy mit bis zu 3 Kohlenstoffatomen substituiert sein kann,
- R: für Wasserstoff oder für Alkyl mit bis zu 6 Kohlenstoffatomen steht, das gegebenenfalls durch eine Gruppe -OR³ und/oder durch Alkoxycarbonyl mit bis zu 4 Kohlenstoffatomen substituiert sein kann, oder für Phenyl steht, das durch Alkyl oder Alkoxy mit bis zu 4 Kohlenstoffatomen substituiert sein kann oder für eine Gruppe der Formeln 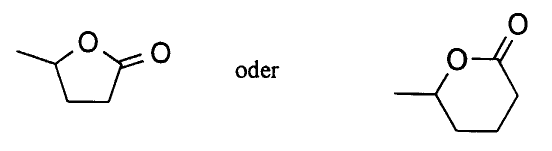 steht oder für Trimethylsilyl-, tert.-Butylsilyl oder Diphenylmethylsilyl steht,
- R¹, R²: für Wasserstoff oder Methyl stehen,
und
- R³: für Wasserstoff oder Alkyl mit bis zu 3 Kohlenstoffatomen oder Trialkylsilyl oder Alkyldiphenylsilyl steht, wobei die Alkylgruppen jeweils bis zu 4 Kohlenstoffatome enthalten.

Als Palladiumverbindungen werden bevorzugt solche mit Palladium in den Oxidationsstufen 0 oder +2 verwendet. Bevorzugt sind hierbei Palladiumhalogenide, Palladiumacetate und Palladiumkomplexe, z.B. Komplexe mit Triphenylphosphin oder Pd(O)dba-Komplexe (dba = Dibenzal-aceton). Besonders bevorzugt sind (PPh₃)PdCl₂, (PPh₃)₄Pd, Pd(dba) oder Pd₂(dba)₃(CHCl₃).

Die Palladiumverbindung wird in der Regel in katalytischen Mengen eingesetzt. Bevorzugt sind dies Mengen von 0,0001 bis 0,2 Äquivalenten, besonders bevorzugt von 0,001 bis 0,01 Äquivalenten, bezogen auf die Verbindungen der allgemeinen Formel (II).

Als Phosphin wird Triphenylphosphin bevorzugt. Die Menge der verwendeten Phosphorverbindung kann z.B. 0 bis 2 Äquivalente, vorzugsweise 0 bis 1 Äquivalente, bezogen auf Palladium, betragen.

Als Basen werden bevorzugt schwache, nicht-nucleophile Basen eingesetzt. Hierzu gehören insbesondere Amine, basische Metalloxide oder -hydroxide sowie die Salze schwacher Säuren. Besonders bevorzugt werden Natriumhydrogencarbonat, Natriumcarbonat, Natriumacetat oder tert. Amine wie Triethylamin oder Diisopropylethylamin eingesetzt.

Die Basen werden z.B. in 1,0 bis 10,0 eq, vorzugsweise in Mengen von 1,0 bis 3,0 eq, bezogen auf Verbindungen der allgemeinen Formel (II) eingesetzt.

Eine der Kupplungskomponenten der allgemeinen Formeln (II) und (III) kann gegebenenfalls in einem Überschuss von bis zu 2 eq, bezogen auf die jeweils andere Komponente, eingesetzt werden.

Als inerte Lösungsmittel eignen sich übliche polare aprotische Lösungsmittel. Hierzu gehört bevorzugt Dimethylformamid, niedere aliphatische Ketone wie beispielsweise Aceton, aliphatische Ether wie beispielsweise tert.-Butylmethylether, cyclische Ether wie Dioxan oder Tetrahydrofuran, aliphatische Mehrfachether wie Diglyme und Ethylenglykoldimethylether oder tertiäre Amine wie Triethylamin oder Diisopropylamin oder niedere Nitrile wie Acetonitril. Ebenso ist es möglich in Gemischen der genannten Lösemittel zu arbeiten. Bei der Verwendung von Aminen als Lösungsmittel kann auf die zusätzliche Verwendung von Basen verzichtet werden.

Das erfindungsgemäße Verfahren kann z.B. in einem Temperaturbereich von 0°C bis 200°C, bevorzugt von 50°C bis 150°C durchgeführt werden.

Im allgemeinen arbeitet man bei Normaldruck, es ist jedoch ebenso möglich unter erhöhtem oder erniedrigten Druck zu arbeiten.

Die Aufarbeitung des Reaktionsgemisches kann z.B. durch Extraktion mit verdünnten Mineralsäuren, übliche Neutralisation z.B. mit wäßrigem Natriumhydrogencarbonat und Trocknung erfolgen.

Bei der Verwendung unlöslicher Basen oder beim Entstehen unlöslicher Reaktionsprodukte von Basen ist es zweckmäßig diese vor der eigentlichen Aufarbeitung abzutrennen, z.B. durch Filtration. Bei der Verwendung von mit Wasser mischbaren Lösungsmitteln kann man diese vorher durch Destillation entfernen und den Rückstand in einem mit Wasser nicht mischbaren Lösungsmittel, wie Methylenchlorid oder tert.-Butylmethylether, aufnehmen.

Die als Ausgangsstoffe eingesetzten Alkine sind bekannt oder können nach bekannten Methoden hergestellt werden. Ebenso sind die eingesetzten Benzylverbindungen bekannt oder können nach bekannten Methoden hergestellt werden.

Die nach dem erfindungsgemäßen Verfahren herstellbaren 3-Arylpropine sind wertvolle Zwischenprodukte zur Herstellung von Leukotrienantagonisten wie Z4-(4-Carboxyphenylthio)-7-[4-(4-phenoxybutoxy)-phenyl]-5-heptensäure (EP-A-0 494 621) in seiner racemischen Form und Analoga.

Das folgende Formelschema erläutert beispielhaft, wie man aus erfindungsgemäß zugänglichen 3-Arylpropinen (Ia) und (Ic) zu der nächsten Stufe (Verbindung (IVa)) auf dem Weg zu dem o.a. Leukotriennantagonisten gelangen kann. 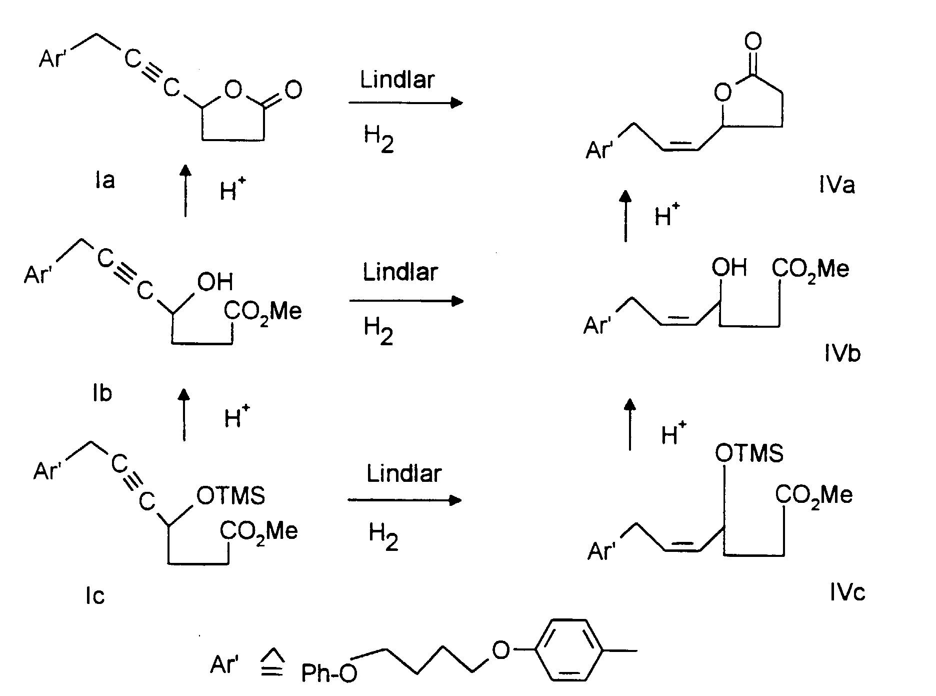 Ib) ist als Zwischenprodukt bereits beschrieben, ebenso IVa) und b) und die Umwandlung von Ib) in IVb) durch Reduktion nach Lindlar (siehe z.B. Methoden der organischen Chemie, 4. Auflage, Band IV/la-Reduktion Teil 1, Seiten 28, 78, 108 und 109 (1980) und die dort genannte Literatur). Analog können nach bekanntem Verfahren Ia) in IVa) und Ic) in IVc) umgewandelt werden.

Die Desilylierung von Ic) oder IVc) zum Alkohol Ib) oder IVb) und auch deren Lactonisierung zu Ia) oder IVa) können nach bekannten Verfahren durch Säurekatalyse oder durch F⁻ erfolgen. Insbesondere kann die Umwandlung Ic) nach Ia) in einem Schritt unter saurer Katalyse (z.B. p-Toluolsulfonsäure in unpolaren Lösungsmitteln wie Methylenchorid oder Toluol) erfolgen.

Die Kupplungsprodukte Ia) bis Ic) können also durch eine geeignete Kombination der bekannten Methoden der Desilylierung, Lactonisierung und Lindlar-Reduktion in die bekannten Zwischenprodukte IVa) oder IVb) umgewandelt werden. Bei Verwendung von enantiomerenreinen Komponenten der allgemeinen Formel (III) können die Zwischenprodukte Ia) bis Ic) und IVa) bis IVc) und damit auch der oben genannte Leukotrienantagonist in enantiomerenreiner Form hergestellt werden.

Die vorliegende Erfindung betrifft weiterhin einige neue 3-Aryl-propine, nämlich 7-(4-(4-Phenoxy-butyloxy)-phenyl)-4-trimethylsilyloxy-hept-5-insäure-methylester, 5-(3-(4-(4-Phenoxy-butyloxy)-phenyl)-prop-1-in-1-yl)-tetrahydrofuran-2-on und 7-(4-(4-Phenoxy-butyloxy)-phenyl)-4-(tetrahydropyran-2-yloxy)-hept-5-insäure-methylester.

### Beispiele

DMF steht für Dimethylformamid; Ph steht für Phenyl; Prozentangaben sind Gewichtsprozente, soweit nicht anders angegeben.

### Beispiel 1

3-(4-Methoxy-phenyl)-1-trimethylsilyl-1-propin

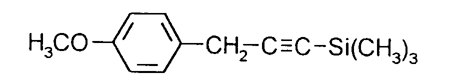

Eine Mischung aus 7,82 g Methoxybenzylchlorid, 9,82 g Trimethylsilylacetylen, 70 ml DMF, 8,4 g Natriumhydrogencarbonat, 348 mg Bistriphenylphosphin-palladiumdichlorid und 131 mg Triphenylphosphin wird 20 Stunden bei 80°C intensiv gerührt. Nach Filtration wird das Filtrat mit 200 ml tert.-Butylmethylether versetzt und zweimal mit Wasser und mit ges. Natriumchlorid-Lösung extrahiert und mit Magnesiumsulfat-Lösung getrocknet. Nach Entfernung des Lösungsmittels wird im Hochvakuum destilliert (Kugelrohr, Kp.: ca. 190°C/0,1 mbar).
Ausbeute: 6,37 g (27 %)
roh: 70 % (GC-Ausbeute)
¹H-NMR (200 MHz, CDCl₃): 0,13 (s, 9H, (CH₃)₃Si), 3,50 (s, 2H, CH₂), 3,71 (s; 3H, CH₃O), 6,78 (d, J = 9Hz; 2H, H_{aromat.}), 7,18 (d, J = 9Hz; 2H, H_{aromat.})
GC/MS/CI (Isobutan) m/z = 219 (100 %, M⁺+H), 203 (10 %), 121 (30 %)

### Beispiel 2

1-(4-Methoxy-phenyl)-2-heptin - LAC 1114

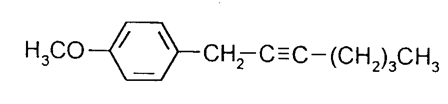

Eine Mischung aus 7,82 g Methoxybenzylchlorid, 4,1 g 1-Hexin, 70 ml DMF, 8,4 g Natriumhydrogencarbonat, 348 mg Bistriphenylphosphin-palladiumdichlorid und 131 mg Triphenylphosphin wird 18 Stunden bei 80°C intensiv gerührt. Nach Filtration wird das Filtrat mit 200 ml tert.-Butylmethylether versetzt und zweimal mit Wasser und mit gesättigter Natriumchlorid-Lösung extrahiert und mit Magnesiumsulfat-Lösung getrocknet. Nach Entfernung des Lösungsmittels wird durch Chromatographie an Kieselgel (Laufmittel Cyclohexan) gereinigt.
Ausbeute: 4,9 g (40 %)
¹H-NMR (200 MHz, CDCl₃): 0,92 (t, J = 7 Hz; 3H, CH₃CH₂), 1,48 (m; 4H, (CH₂)₂CH₃), 2,22 (m; 2H, C=CCH₂), 3,52 (s; 2H, ArCH₂), 3,79 (s; 3H, OCH₃), 7,25 (d, J = 9Hz; 2H, H_{aromat.}), 7,25 (d, J = 9Hz; 2H, H_{aromat.})
GC/MS/CI (Isobutan) m/z = 259 (50 %, M⁺+C₄H₉), 202 (100 %, M⁺-H), 121 (40 %)

### Beispiel 3

3-(4-Methoxy-phenyl)-1-phenyl-1-propin

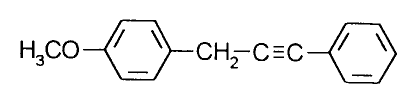

Eine Mischung aus 5 g Methoxybenzylchlorid, 3,5 g Phenylacetylen, 50 ml DMF, 13 g Natriumhydrogencarbonat, 150 mg Bistriphenylphosphin-palladiumdichlorid und 50 mg Triphenylphosphin wird 5,5 Stunden bei 80°C intensiv gerührt. Nach Filtration wird das Filtrat mit 200 ml tert.-Butylmethylether versetzt und zweimal mit Wasser und mit gesättigter Natriumchlorid-Lösung extrahiert und mit Magnesiumsulfat-Lösung getrocknet. Nach Entfernung des Lösungsmittels wird durch Chromatographie an Kieselgel (Laufmittel Cyclohexan) gereinigt.
Ausbeute: 5,1 g (72 %)
¹H-NMR (200 MHz, CDCl₃): 3,77 (s; 2H, ArCH₂), 3,79 (s; 3H, OCH₃), 6,88 (d, J = 9Hz; 2H, H_{aromat.}), 7,2 - 7,5 (m; 7H, H_{aromat.})
GC/MS/CI (Isobutan) m/z = 279 (40 %, M⁺-C₄H₉), 223 (100 %, M⁺+H), 121 (30 %), 115 (20 %)

### Beispiel 4

3-(4-Methoxy-phenyl)-1-phenyl-1-propin - LAC 1069-7

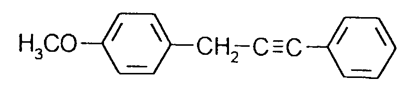

Eine Mischung aus 5 g Methoxybenzylchlorid, 3,75 g Phenylacetylen, 50 ml THF, 2,9 g Natriumacetat, 125 mg Bistriphenylphosphin-palladiumdichlorid und 50 mg Triphenylphosphin wird 16 Stunden unter Rückfluss intensiv gerührt. Nach Filtration wird das Filtrat eingeengt und destilliert.
Ausbeute: 3,3 g (57 %)

### Beispiel 5

3-(4-(4-Phenoxy-butyloxy)-phenyl)-1-phenyl-1-propin

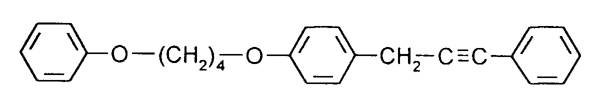

Eine Mischung aus 2 g (4-(4-Phenoxy-butyloxy)-phenyl)-methylchlorid, 1,4 g Phenylacetylen, 20 ml THF, 1,2 g NaHCO₃, 50 mg (PPh₃)₂PdCl₂ und 25 mg PPh₃ wird 16 Stunden unter Rückfluß gekocht und kalt filtriert. Das Filtrat wird eingeengt und durch Chromatographie an Kieselgel gereinigt.
Ausbeute: 1,20 g (49 %)
GC/MS/CI: m/z = 357 (100 % / M⁺+1)
¹H-NMR (CDCl₃, 200 MHz): 1,98 (m, 4H; CH₂(CH₂)₂), 3,77 (s; 2H, ArCH₂), 4,02 (m; 4H, OCH₂), 6,8 bis 7,0 (m; 2H, H_{aromat.}), 7,2 bis 7,35 (m; 7H, H_{aromat.}), 7,4 bis 7,5 (m; 5H, H_{aromat.})

Das Edukt 4-(4-Phenoxy-butyloxy)-phenylmethylchlorid wurde wie folgt hergestellt: Durch eine Lösung von 7g 4-(4-Phenoxy-butyloxy)-phenylmethanol (EP 0 410 241) in 18 ml Chloroform wird bei Raumtemperatur 30 min HCI-Gas eingeleitet. Anschließend wird einrotiert (Ausbeute quantitativ).
¹H-NMR (200 MHz, [D₆]-DMSO): 1,85 (m; 4H), 4,05 (m; 4H), 4.70 (s; 2H) 6.80 - 6.97 (m; 5H), 7.20 - 7.38 (m; 4H)

### Beispiel 6

7-(4-Methoxy-phenyl)-4-trimethylsilyloxy-hept-5-insäure-methylester

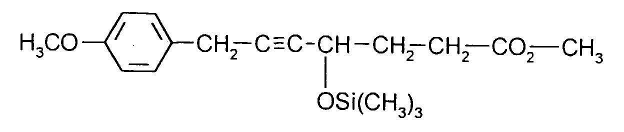

Eine Mischung aus 1,4 g 4-Methoxy-benzylchlorid, 2 g 4-Trimethylsilyloxy-hex-5-insäuremethylester, 14 ml Dioxan, 2,4 g NaHCO₃, 0,4 g (PPh₃)₂PdCl₂ und 200 g PPh₃ wird 7 Stunden unter Rückfluß gekocht und kalt filtriert. Das Filtrat wird eingeengt und durch Chromatographie an Kieselgel gereinigt.
Ausbeute: 1,4 g (45 %)
GC/MS/CI: m/z = 335 (100 % / M⁺+1)
¹H-NMR (200 MHz, CDCl₃): 0,0 (s; 9H, (CH)₃Si), 1,84 (q, J = 7Hz, 2H CH₂COO), 2,32 (t; J = 7Hz, 2H, OCHCH₂), 3,4 (d, J = 2Hz, 2H, ArCH₂), 3,53, 3,63 (2x s; 2x 3H, OMe), 4,32 (tt, J = 6Hz, J = 2Hz; 1H, CHOSi), 6,70 (d, J = 8Hz, 2H, H_{aromat.}), 7,08 (d, J = 8Hz, 2H, H_{aromat.})

Das Edukt wurde wie folgt hergestellt:
4-Hydroxy-5-hexinsäuremethylester (J. Med. Chem. 32, 863 (1989)) wurde nach der üblichen Methode mit je 1.2 eq Trimethylsilylchlorid/Triethylamin in Methylenchlorid 1.5 Stunden bei Raumtemperatur silyliert und destilliert (Kp.: 62°C / 0.08 mbar, Ausbeute 86 %).

### Beispiel 7

1,3-Diphenyl-1-propin

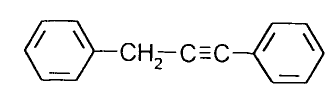

Eine Mischung aus 5 g Benzylchlorid, 4,5 g Phenylacetylen, 50 ml Triethylamin, 0,25 g (PPh₃)₂PdCl₂ und 100 g PPh₃ wird 4 Stunden unter Rückfluß gekocht und weitgehend vom Lösungsmittel befreit. Der Rückstand wird mit tert.-Butylmethylether und 1N HCl aufgenommen. Die organische Phase wird nochmal mit 1N HCl und gesättigter NaCl-Lösung gewaschen, mit MgSO₄ getrocknet, eingeengt und destilliert (Kp 145°C, 0,1 mbar).
Ausbeute: 5,43 g (66 %)
GC/MS/CI: e/m = 335 (100 % / M⁺+1)
¹H-NMR (200 MHz, CDCl₃): 3,81 (s, 2H, CH₂), 7,20 - 7,55 (m, 10H, H_{aromat.})

### Beispiel 8

3-(4-Methoxy-phenyl)-1-phenyl-1-propin (mit PdCl₂)

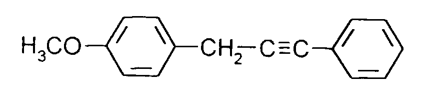

Eine Mischung aus 7,8 g Methoxybenzylchlorid, 5,1 g Phenylacetylen, 70 ml DMF, 8,4 g Natriumhydrogencarbonat und 89 mg Palladiumdichlorid wird 8 Stunden bei 80°C intensiv gerührt. Nach Filtration wird das Filtrat mit 200 ml tert.-Butylmethylether versetzt und zweimal mit Wasser und mit gesättigter Natriumchlorid-Lösung extrahiert und mit Magnesiumsulfat-Lösung getrocknet. Nach Entfernung des Lösungsmittels wird durch Chromatographie an Kieselgel (Laufmittel Cyclohexan) gereinigt.
Ausbeute: 6,1 g (56 %)

### Beispiel 9

5-(3-(4-(4-Phenoxybutyloxy)-phenyl)-prop-1-in-1-yl)-tetrahydrofuran-2-on

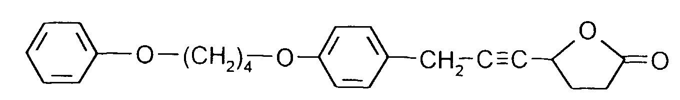

Eine Mischung aus 275 mg 5-Ethinyl-tetrahydrofuran-2-on (U. Macksell, J. Med. Chem. 33, 863 (1989)), 1,30 g 4-(4-Phenoxybutyloxy)-benzylchlorid, 15 ml Dioxan, 760 mg Natriumhydrogencarbonat, 116 mg Bis(Triphenylphosphin)-palladiumdichlorid und 36 mg Triphenylphosphin wird 24 Stunden unter Rückfluß intensiv gerührt. Nach 3 und 6 Stunden werden je 275 mg 5-Ethinyl-tetrahydrofuran-2-on zugegeben. Nach Filtration wird das Filtrat mit 200 ml tert.-Butylmethylether versetzt und zweimal mit Wasser und mit ges. Natriumchlorid-Lösung extrahiert und mit Magnesiumsulfat-Lösung getrocknet. Nach Entfernung des Lösungsmittels und chromatographischer Reinigung verbleiben 813 mg Produkt (50 % Ausbeute bezogen auf 4-(4-Phenoxybutyloxy)-benzylchlorid).
¹H-NMR (200 MHz, CDCl₃) : 1,95 (m; 4H, C-(CH₂)₂-C, 2.2 - 2.7 (m; 4H, CC(CH₂)CO₂), 3.55 (d, J = 3Hz; 2H, ArCH₂), 3.85 (m; 4H, OCH₂), 5.15 (m; 1H, OCH), 6.75 - 6.95 (m; 5H), 7.15 - 7.30 (m; 4H).

### Beispiel 10

3-(4-Methoxy-phenyl)-1-phenyl-1-propin mit Na(CO₃)₂

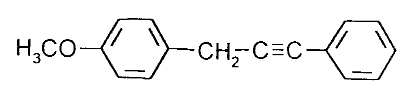

Eine Mischung aus 7,8 g Methoxybenzylchlorid, 5,1 g Phenylacetylen, 70 ml DMF, 10,6 g Natriumcarbonat, 348 mg Bis(Triphenylphosphin)-palladium-dichlorid und 131 mg Triphenylphosphin wird 4 Stunden bei 80°C intensiv gerührt. Nach Filtration wird das Filtrat mit 200 ml tert.-Butylmethylether versetzt und zweimal mit Wasser und mit gesättigter Natriumchlorid-Lösung extrahiert und mit Magnesiumsulfat-Lösung getrocknet. Nach Entfernung des Lösungsmittels ergibt die Chromatographie (Laufmittel Cyclohexan) 7,2 g (65 % Ausbeute) Produkt.

### Beispiel 11

7-(4-(4-(Phenoxy-butyloxy)-phenyl)-4-trimethylsilyloxy-hept-5-insäure-methylester

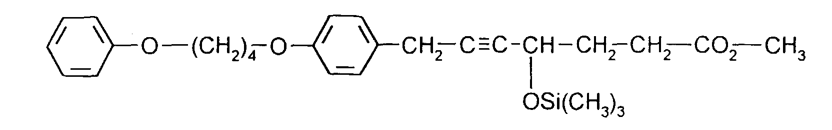

Eine Mischung aus 1,0 g 4-(4-Phenoxy-butyloxy)benzylchlorid, 325 mg 4-Trimethylsilyloxy-hex-5-insäuremethylester, 15 ml Dioxan, 1.17 g NaHCO₃, 0,2g (PPh₃)₂ PdCl₂ und 100 mg PPh₃ wird 5 Stunden unter Rückfluß gekocht. Nach 1, 2 und 3,5 Stunden werden je 325 mg 4-Trimethylsilyloxy-hex-5-insäuremethylester zugegeben. Die erkaltete Mischung wird filtriert. Das Filtrat wird eingeengt und durch Chromatographie (15 % Essigester in Cyclohexan) an Kieselgel gereinigt. Ausbeute: 1,15 g (72 %)
¹H-NMR (200 MHz, CDCl₃) : 0.0 (s; 9H, Si (CH₃)₃), 1,8 (m; 6H, C-(CH₂)₂-C + C-CH₂-C), 2,32 (t, J = 7.5 Hz; 2H, CH₂CO₂), 3.39 (d, J = 3Hz; 1H, ArCH₂), 3.52 (s; 3H, OCH₃), 3.85 (m; 4H, OCH₂), 4.31 (dt, J_{d} = 3Hz; Jₜ = 7Hz; 1H, OCH), 6.65-6.80 (m; 5H), 7.02 - 7.18 (m; 4H)

### Beispiel 12

7-(4-(4-(Phenoxy-butyloxy)-phenyl)-4-hydroxy-hept-5-insäure-methylester

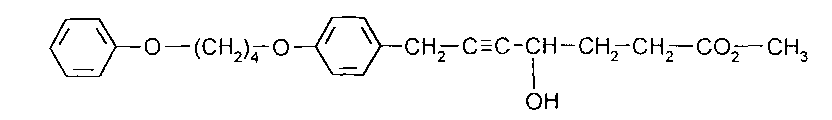

Es wurde verfahren wie im Beispiel 9, nur wurde anstatt 5-Ethinyl-tetrahydrofuran-2-on eine äquimolare Menge 4-Hydroxy-5-hexinsäuremethylester verwendet.
Ausbeute: 36 %
¹H-NMR (500 MHz, CDCl₃) : 1,96 (m; 4H, C-(CH₂)₂-C), 2.04 (q, J = 7Hz; 2H, C(CH₂)C), 2.54 (m; 2H, CH₂CO), 3.56 (d, J = 2Hz; 1H, ArCH₂), 3.68 (s; 3H, OCH₃), 4.02 (m; 4H, 2x(OCH₂)), 4.51 (t, J = 7Hz; 1H, OCH), 6.80 - 6.95 (m; 5H), 7.20 - 7.30 (m; 4H)

### Beispiel 13

5-(3-(4-(4-(Phenoxybutyloxy)-phenyl)-prop-1-in-1-yl)-tetrahydrofuran-2-on aus 7-(4-(4-Phenoxybutyloxy-)-phenyl)-4-trimethylsilyloxy-hept-5-in-säuremethylester

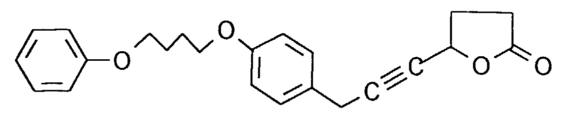

Eine Lösung von 7.67 g 7-(4-(4-Phenoxybutyloxy-)-phenyl)-4-trimethylsilyloxy-hept-5-in-säuremethylester und 770 mg p-Toluolsulfonsäure in 77 ml Methylenchlorid und 0,25 ml Wasser wird 6 Stunden bei Raumtemperatur gerührt. Es werden 50 mg NaHCO₃ zugegeben, 5 min gerührt, filtriert, mit Wasser und gesättigter NaCl-Lsg. extrahiert und mit MgSO₄ getrocknet. Nach Chromatographie (35 % Essigester in Cyclohexan) bleiben 3,4 g (54 %) Produkt.

### Beispiel 14

5-(3-(4-(4-Phenoxybutyloxy)-phenyl)-prop-1-in-1-yl)-tetrahydrofuran-2-on aus 3-(4-(4-Phenoxybutyloxy-)-phenyl)-4-hydroxy-hept-5-in-säuremethylester

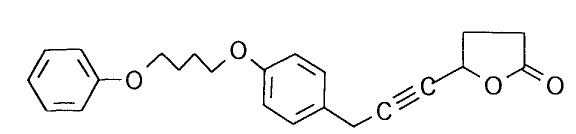

Ausgehend von der Hydroxyverbindung wurde verfahren wie im Beispiel 13. Ausbeute 65 %.

### Beispiel 15

(Z)-5-(3-(4-(4-Phenoxybutyloxy)-phenyl)-prop-1-en-1-yl)-tetrahydrofuran-2-on

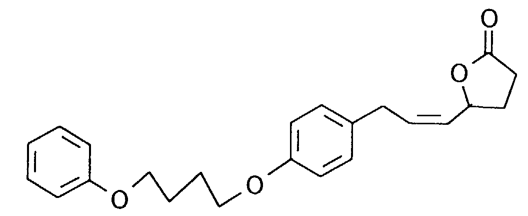

1.02 g 5-(3-(4-(4-Phenoxybutyloxy)-phenyl)-prop-1-in-1-yl)-tetrahydrofuran-2-on in 28 ml Essigester wurde mit 252 mg Pd auf CaCO₃ (5%) und 5,6 ml Chinolin versetzt und bei 1 atm Wasserstoff bei Raumtemperatur hydriert. Die Reduktion wird nach Aufnahme von einem Equivalent Wasserstoff abgebrochen. Nach Filtration wird mit MgSO₄ getrocknet und eingeengt.
Ausbeute: quantitativ
¹H-NMR (200 MHz, CDCl₃): 1,9-2,2 (m; 8H), 3,4 (m, 2H), 4.01 (m, 4H), 5,35 (m, 1H), 5,58 (6, J=10 Hz; 1H), 5.82 (m; 1H), 6,7-7,3 (m, 9H).

### Beispiel 16

7-(4-(4-Phenoxy-butyloxy)-phenyl)-4-(tetrahydropyran-2-yloxy)-5-heptincarbonsäuremethylester

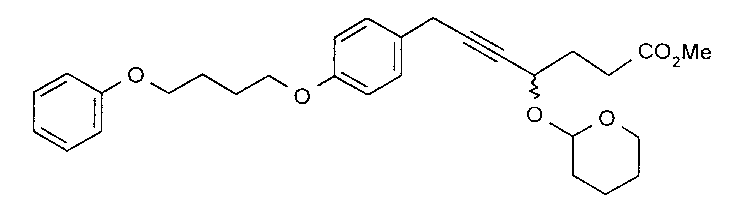

Eine Mischung aus 13,06 g 4-(4-Phenoxybutyloxy)-benzylchlorid in 245 ml absolutem Dioxan mit 11,5 g Natriumhydrogencarbonat, 0,915 g Bis(Triphenylphosphin)-palladium(II)chlorid und 0,457 g Triphenylphosphin wird unter Argon zum Rückfluß erhitzt.

Es wird innerhalb von 8 Stunden 12,06 g 4-(Tetrahydropyran-2-yloxy)-5-hexincarbonsäuremethylester bei 140°C zugetropft und 15 min bei 140°C nachrührt.

Zur Aufarbeitung wird die erkaltete Mischung abfiltriert und das Filtrat eingeengt. Das Rohprodukt wird in Tert.-butyl-methylether aufgenommen und mit Wasser gewaschen. Die organische Phase wird mit MgSO₄ getrocknet, eingeengt und über Nacht im Hochvakuum bei Raumtemperatur getrocknet. Man erhält 21,89 g (104 %) Rohprodukt.
¹H-NMR (CDCl₃, 200 MHz): δ = 1,5-1,9 (m; 6H, (CH₂)₃), 1,98 (br, s, 4H; (OCH₂(CH₂)₂), 2.09 (q, 7 Hz; 2H CH₂CH₂COOMe), 2,54 (t., J = 7 Hz; 2H, CH₂COOMe), 3,4 - 3,8 (m; 2H, CH₂O), 3.56 (s, 2H, ArCH₂); 3,68 (s; 3H, OCH₃), 4,0 (br, s; 4H PhOCH₂), 4,46, 4,53 (2xt, J = 7 Hz; 1H, C≡CCHO), 4,76, 5,02 (2x, br, s; 1H; OCHO), 6,8-7,0 (m; 5H H_{aromat.}), 7,2-7,35 (m; 5H, H_{aromat.}).

Das Edukt wird wie folgt hergestellt: 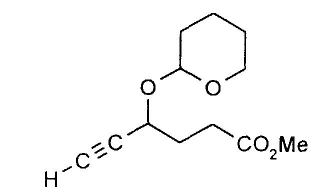 4-(Tetrahydropyran-2-yloxy)-5-hexinsäuremethylester

Zu einer Mischung aus 79 g 4-Hydroxy-5-hexincarbonsäuremethylester und 64 g Dihydropyran wird bei 0°C 0,56 ml konz. Salzsäure zugetropft. Anschließend wird 18 Stunden bei Raumtemperatur gerührt.

Zur Aufarbeitung wird 1 g Kaliumhydroxid (fest) zugegeben, 15 min gerührt, filtriert und im Hochvakuum über eine Kolonne destilliert. Die Destillation (Kp.: 83 - 88°C / 0,08 mbar) ergibt 111,6 g (88,2 % d. Th.).
¹H-NMR (CDCl₃, 200 MHz): δ = 1,5 - 1,9 (m, 6H), 2,08 (q, 7 Hz; 2H, CH₂CH₂COOMe), 2,55 (t, J = 7 Hz; 2H, CH₂COOMe), 2,41 (d, J = 2 Hz; 1H, C=CH), 3,4 - 3,8 (m; 2H, CH₂O), 3,68 (s; 3H, OCH₃), 4,38, 4,48 (2xdt, J_{d}=2Hz, Jₜ= 7 Hz; 1H, C=CCHO), 4,75, 4,98 (2x br, s; 1H; OCHO)

### Beispiel 17

5-(3-(4-(4-Phenoxybutyloxy)-phenyl)-prop-1-in-1-yl)-tetrahydrofuran-2-on aus 7-(4-(4-Phenoxy-butyloxy)-phenyl-4-(tetrahydropyran-2-yloxy)-5-heptinsäuremethylester

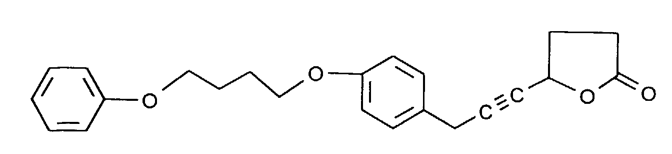

Eine Mischung aus 21,8 g 7-(4-(4-Phenoxy-butyloxyphenyl)-(4-tetrahydropyran-2-yloxy)-5-heptincarbonsäuremethylester, 330 ml Methylenchlorid, 2,6 g p-Toluolsulfonsäure und 0,86 ml Wasser wird 2,5 Stunden bei Raumtemperatur gerührt.

Zur Aufarbeitung wird die Lösung mit 200 ml gesättigter Natriumhydrogencarbonatlösung ausgeschüttelt.

Die organische Phase wird mit NaCl-Lösung gewaschen, mit MgSO₄ getrocknet und eingeengt.

Man erhält 19,85 g Rohprodukt, welches mit 150 ml Methanol über Nacht ausgerührt wird.

Nach Absaugen des Feststoffes wird die Mutterlauge auf die Hälfte eingeengt, im Kühlschrank über Nacht gelagert und abgesaugt. Die vereinigten Feststoffe ergeben 12,2 g (75 % d. Th., bezogen auf 4-(4-Phenoxy-butyloxy)-benzylchlorid Produkt.

### Beispiel 18

7-(4-(4-Phenoxy-butyloxy)-phenyl-4-(acetyloxy)-5-heptinsäuremethylester

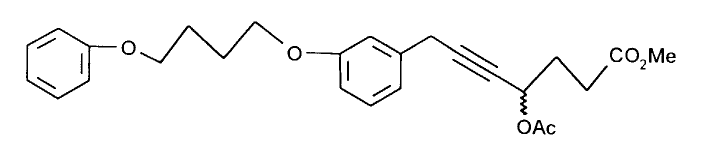

Eine Mischung aus 4 g 4-(4-Phenoxybutyloxy)-benzylchlorid in 75 ml absolutem Dioxan mit 3,5 g NaHCO₃, 280 mg Bis-(Triphenylphosphin)palladium(II)chlorid und 140 mg Triphenylphosphin wird unter Argon unter Rückfluß erhitzt. 3,01 g 4-Acetyloxy-5-hexincarbonsäuremethylester wird in 4 Portionen innerhalb von 4,5 Stunden zugegeben und es wird noch weitere 3,5 Stunden unter Rückfluß gerührt.

Zur Aufarbeitung wird die erkaltete Mischung abfiltriert und das Filtrat eingeengt. Das Rohprodukt wird in Tert.-butyl-methylether aufgenommen und mit Wasser gewaschen. Die organische Phase wird mit MgSO₄ getrocknet, eingeengt und über eine Kieselgelschicht (Laufmittel: Essigester/Cyclohexan 1:5) filtriert. Man erhält 1,95 g (32 % d. Th.) Produkt.
¹H-NMR (CDCl₃, 200 MHz): δ = 1,97 (br, s, 4H; (OCH₂(CH₂)₂), 2,12 (q, 7 Hz; 2H, CH₂CH₂COOMe), 2.50 (t, J = 7Hz; 2H, CH₂COOMe), 3,57 (d, J = 2 Hz; 2H, ArCH₂), 3,70 (s; 3H, OCH₃), 4,02 (br s; 4H PhOCH₂), 549 (tt, J = 7Hz, J = 2 HZ; 1H, C=CCHO), 6,8 - 7,0 (m; 5H, H_{aromat.}), 7,2 - 7,35 (m; 5H, Hₐᵣₒₘₐₜ).

Das Edukt wird wie folgt hergestellt:

4-Acetyloxy-5-hexinsäuremethylester 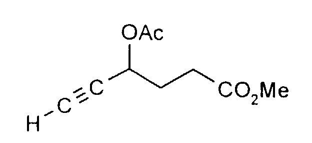 Eine Mischung aus 25 g 4-Hydroxy-5-hexincarbonsäuremethylester, 17,5 ml Essigsäureanhydrid und 180 ml Pyridin werden 3 Stunden auf Raumtemperatur erhitzt.

Man gibt auf Eis, extrahiert mit Tert.-butyl-methylether. Die organische Phase wird mit 1 N HCl gewaschen, mit MgSO₄ getrocknet und eingeengt.

Das Rohprodukt wird im Vakuum (76°C / 0,15 mbar) destilliert. Man erhält 26,93 g (83 % d. Th.) Produkt.

## Patentansprüche

1. Verfahren zur Herstellung von 3-Aryl-propinen der allgemeinen Formel (I) 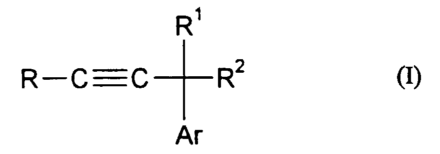 in welcher
Ar
- für Aryl mit 6 bis 10 Kohlenstoffatomen steht, das gegebenenfalls durch Alkyl, Alkylsulfonyl oder Alkoxycarbonyl mit bis zu 10 Kohlenstoffatomen oder durch Alkoxy mit bis zu 8 Kohlenstoffatomen substituiert sein kann, wobei die Alkoxygruppen wiederum durch Phenoxy oder Alkoxy mit bis zu 6 Kohlenstoffatomen untersubstituiert sein können oder
- für einen 5- bis 6-gliedrigen, gegebenenfalls Benzo-kondensierten heterocyclischen Ring steht, der als Heteroatome bis zu 2 Stickstoff-, Sauerstoff- und/oder Schwefelatome enthalten kann und der gegebenenfalls durch Alkyl, Alkoxy oder Alkoxycarbonyl mit bis zu 6 Kohlenstoffatomen oder durch Phenyl substituiert sein kann,
R
- für Wasserstoff oder Alkyl mit bis zu 10 Kohlenstoffatomen steht, das gegebenenfalls durch eine Gruppe -OR³, -NHR³ oder -NR³₂ und/oder Alkoxycarbonyl mit bis zu 6 Kohlenstoffatomen substituiert sein kann oder
- für Aryl mit 6 bis 10 Kohlenstoffatomen steht, das gegebenenfalls durch Alkyl, Alkoxy oder Alkoxycarbonyl mit bis zu 8 Kohlenstoffatomen substituiert ist oder
- für eine Trialkylsilyl- oder eine Alkyldiphenylsilyl-Gruppe steht, wobei die Alkylgruppen bis zu 6 Kohlenstoffatomen enthalten können oder
- für eine Gruppe der Formeln 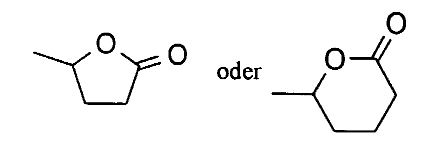 steht,
R¹, R² gleich oder verschieden sind und
- für Wasserstoff, Alkyl mit bis zu 6 Kohlenstoffatomen oder Aryl mit 6 bis 10 Kohlenstoffatomen stehen oder
- gemeinsam mit dem dazwischen liegenden Kohlenstoffatom einen Cyclopentyl- oder Cyclohexylring bilden
und
R³ für Wasserstoff, Alkyl mit bis zu 6 Kohlenstoffatomen, Acyl mit bis zu 6 Kohlenstoffatomen, Benzoyl, Tetrahydropyranyl, Trialkylsilyl oder Alkyldiphenylsilyl steht, wobei die Alkylgruppen hier jeweils bis zu 6 Kohlenstoffatomen enthalten,
dadurch gekennzeichnet, daß man
Benzylverbindungen der allgemeinen Formel (II) 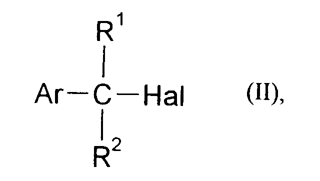 in welcher
Ar, R¹ und R² die bei Formel (I) angegebene Bedeutung haben und
Hal für Chlor oder Brom steht,
mit Alkinen der allgemeinen Formel (III)
H―C≡C―R (III)
in der R die bei Formel (I) angegebene Bedeutung haben und
in Gegenwart einer Palladium-Verbindung, gegebenenfalls in Anwesenheit von Phosphinen, mit Hilfe von schwachen Basen in inerten Lösungsmitteln umsetzt.

2. Verfahren nach Anspruch 1, dadurch gekennzeichnet, daß man Palladiumverbindungen mit Palladium in den Oxidationsstufen 0 oder +2 verwendet.

3. Verfahren nach Anspruch 1 bis 2, dadurch gekennzeichnet, daß man Palladiumhalogenide, Palladiumacetate oder Palladiumkomplexe verwendet.

4. Verfahren nach Anspruch 1 bis 3, dadurch gekennzeichnet, daß man die Palladiumverbindung in Mengen von 0,0001 bis 0,2 Äquivalenten, bezogen auf die Verbindungen der Formel (II), einsetzt.

5. Verfahren nach Anspruch 1 bis 4, dadurch gekennzeichnet, daß man als Basen schwache, nicht nukleophile Basen einsetzt.

6. Verfahren nach Anspruch 1 bis 5, dadurch gekennzeichnet, daß man 3-Arylpropine der allgemeinen Formel (I) herstellt, worin
Ar
- für Phenyl oder Naphthyl steht, das gegebenenfalls durch Alkyl oder Alkoxycarbonyl mit bis zu 8 Kohlenstoffatomen oder durch Alkoxy mit bis zu 6 Kohlenstoffatomen substituiert sein kann, wobei die Alkoxygruppen wiederum durch Phenoxy oder Alkoxy mit bis zu 4 Kohlenstoffatomen substituiert sein können oder
- für Thienyl, Furyl, Pyridyl, Pyrimidyl, Pyrazinyl, Chinolyl, Isochinolyl, Benzofuryl, Imidazolyl, Benzimidazolyl oder Indolyl steht, die gegebenenfalls durch Alkyl oder Alkoxy mit bis zu 4 Kohlenstoffatomen oder durch Phenyl substituiert sind,
R
- für Wasserstoff oder Alkyl mit bis zu 8 Kohlenstoffatomen steht, das gegebenenfalls durch eine Gruppe -OR³ und/oder Alkoxycarbonyl mit bis zu 4 Kohlenstoffatomen substituiert ist oder
- für Phenyl oder Naphthyl steht, das gegebenenfalls durch Alkyl oder Alkoxy mit bis zu 6 Kohlenstoffatomen substituiert ist oder
- für eine Trialkylsilyl- oder Alkyldiphenylsilylgruppe steht, wobei die Alkylgruppen bis zu 4 Kohlenstoffatomen enthalten können oder
- für eine Gruppe der Formeln 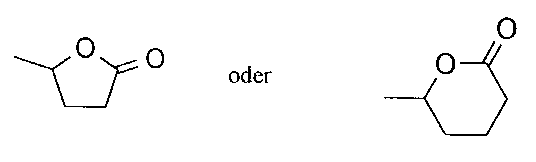 steht,
R¹, R² gleich oder verschieden sind und
- für Wasserstoff, Alkyl mit bis zu 4 Kohlenstoffatomen oder für Phenyl stehen
und
R³ für Wasserstoff, Alkyl mit bis zu 4 Kohlenstoffatomen, Acetyl oder Benzoyl steht oder für Tetrahydropyranyl, Trialkylsilyl oder Alkyldiphenylsilyl steht, wobei die Alkylgruppen jeweils bis zu 4 Kohlenstoffatome enthalten.

7. Verfahren nach Anspruch 1 bis 6, dadurch gekennzeichnet, daß man 3-Arylpropine der allgemeinen Formel (I) herstellt, worin
Ar für Phenyl steht, das gegebenenfalls durch Alkyl mit bis zu 4 Kohlenstoffatomen oder Alkoxy mit bis zu 6 Kohlenstoffatomen substituiert ist, wobei die Alkoxygruppe wiederum durch Phenoxy oder Alkoxy mit bis zu 3 Kohlenstoffatomen substituiert sein kann,
R für Wasserstoff oder für Alkyl mit bis zu 6 Kohlenstoffatomen steht, das gegebenenfalls durch eine Gruppe -OR³ und/oder durch Alkoxycarbonyl mit bis zu 4 Kohlenstoffatomen substituiert sein kann, oder für Phenyl steht, das durch Alkyl oder Alkoxy mit bis zu 4 Kohlenstoffatomen substituiert sein kann oder für eine Gruppe der Formeln 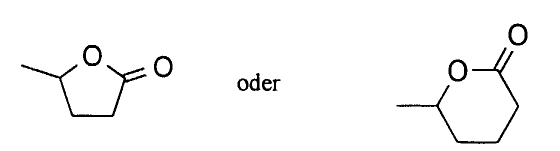 steht oder für Trimethylsilyl-, tert.-Butylsilyl oder Diphenylmethylsilyl steht,
R¹, R² für Wasserstoff oder Methyl stehen,
und
R³ für Wasserstoff oder Alkyl mit bis zu 3 Kohlenstoffatomen oder für eine Trialkylsilyl oder Dialkyldiphenylsilylgruppe steht, wobei die Alkylgruppen jeweils bis zu 4 Kohlenstoffatome enthalten können.

8. 7-(4-(4-Phenoxy-butyloxy)-phenyl)-4-trimethylsilyloxy-hept-5-insäuremethylester.

9. 5-(3-(4-(4-Phenoxy-butyloxy)-phenyl)-prop-1-in-1-yl)-tetrahydrofuran-2-on.

10. 7-(4-(4-Phenoxy-butyloxy)-phenyl)-4-(tetrahydropyran-2-yloxy)-hept-5-insäuremethylester.

## Claims

1. Process for preparing 3-aryl-propines of the general formula (1) 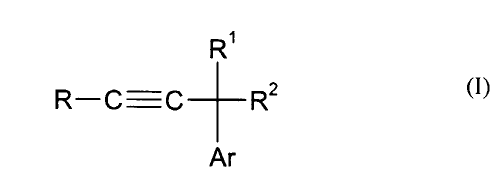 where
Ar represents aryl having from 6 to 10 carbon atoms, which may optionally be substituted by alkyl, alkylsulphonyl or alkoxycarbonyl having up to 10 carbon atoms or by alkoxy having up to 8 carbon atoms, where the alkoxy groups can in turn be substituted by phenoxy or alkoxy having up to 6 carbon atoms, or
represents a 5- to 6-membered, optionally benzo-fused heterocyclic ring which can contain up to 2 nitrogen, oxygen and/or sulphur atoms as heteroatoms and which can optionally be substituted by alkyl, alkoxy or alkoxycarbonyl having up to 6 carbon atoms or by phenyl,
R represents hydrogen or alkyl having up to 10 carbon atoms, which may optionally be substituted by a group -OR³, -NHR³ or -NR³₂ and/or alkoxycarbonyl having up to 6 carbon atoms, or
represents aryl having from 6 to 10 carbon atoms, which is optionally substituted by alkyl, alkoxy or alkoxycarbonyl having up to 8 carbon atoms, or
represents a trialkylsilyl or an alkyldiphenylsilyl group, where the alkyl groups can contain up to 6 carbon atoms, or
represents a group of the formula 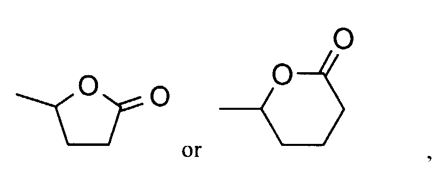 where
R¹, R² are identical or different and
represent hydrogen, alkyl having up to 6 carbon atoms or aryl having from 6 to 10 carbon atoms or
together with the carbon atom between them form a cyclopentyl or cyclohexyl ring
and
R³ represents hydrogen, alkyl having up to 6 carbon atoms, acyl having up to 6 carbon atoms, benzoyl, tetrahydropyranyl, trialkylsilyl or alkyldiphenylsilyl, where the alkyl groups here each contain up to 6 carbon atoms,
characterized in that benzyl compounds of the general formula (Il) 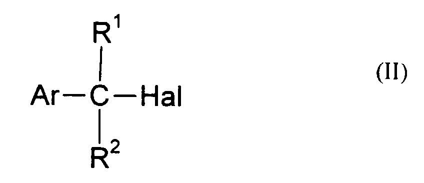 where
Ar, R' and R² have the meaning given in formula (I) and
Hal represents chlorine or bromine,
are reacted with alkines of the general formula (III)
H-C≡C-R (III)
in which R has the meaning given in formula (I) and in the presence of a palladium compound, optionally in the presence of phosphines, with the aid of weak bases in inert solvents.

2. Process according to Claim 1, characterized in that the palladium compounds used contain palladium in the oxidation state 0 or +2.

3. Process according to Claim 1 to 2, characterized in that palladium halides, palladium acetates or palladium complexes are used.

4. Process according to Claim 1 to 3, characterized in that the palladium compound is used in amounts of from 0.0001 to 0.2 equivalents, based on the compounds of the formula (II).

5. Process according to Claim 1 to 4, characterized in that bases used are weak, non-nucleophilic bases.

6. Process according to Claim 1 to 5, characterized in that the 3-aryl-propines prepared have the general formula (I) in which
Ar represents phenyl or naphthyl which can optionally be substituted by alkyl or alkoxycarbonyl having up to 8 carbon atoms or by alkoxy having up to 6 carbon atoms, where the alkoxy groups can in turn be substituted by phenoxy or alkoxy having up to 4 carbon atoms, or
represents thienyl, furyl, pyridyl, pyrimidyl, pyrazinyl, quinolyl, isoquinolyl, benzofuryl, imidazolyl, benzimidazolyl or indolyl which are optionally substituted by alkyl or alkoxy having up to 4 carbon atoms or by phenyl,
R represents hydrogen or alkyl having up to 8 carbon atoms, which is optionally substituted by a group -OR³ and/or alkoxycarbonyl having up to 4 carbon atoms, or
represents phenyl or naphthyl which is optionally substituted by alkyl or alkoxy having up to 6 carbon atoms, or
represents a trialkylsilyl or alkyldiphenylsilyl group, where the alkyl groups can contain up to 4 carbon atoms, or
represents a group of the formula 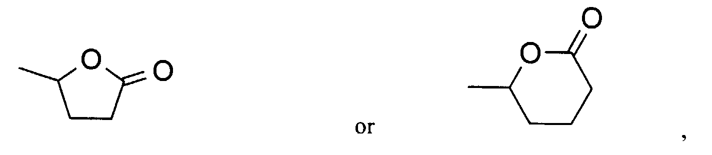
R¹, R² are identical or different and
represent hydrogen, alkyl having up to 4 carbon atoms or represent phenyl
and
R³ represents hydrogen, alkyl having up to 4 carbon atoms, acetyl or benzoyl or represents tetrahydropyranyl, trialkylsilyl or alkyldiphenylsilyl, where the alkyl groups each contain up to 4 carbon atoms.

7. Process according to Claim 1 to 6, characterized in that 3-aryl-propines prepared have the general formula (I) in which
Ar represents phenyl which is optionally substituted by alkyl having up to 4 carbon atoms or alkoxy having up to 6 carbon atoms, where the alkoxy group can in turn be substituted by phenoxy or alkoxy having up to 3 carbon atoms,
R represents hydrogen or represents alkyl having up to 6 carbon atoms, which can optionally be substituted by a group -OR³ and/or alkoxycarbonyl having up to 4 carbon atoms, or represents phenyl which can be substituted by alkyl or alkoxy having up to 4 carbon atoms or represents a group of the formula 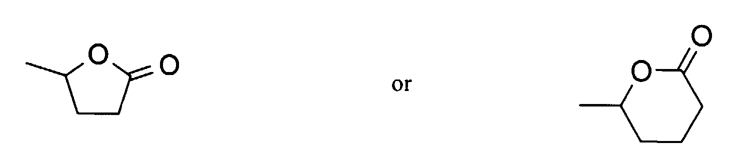 or represents trimethylsilyl, tert-butylsilyl or diphenylmethylsilyl,
R¹, R² represent hydrogen or methyl,
and
R³ represents hydrogen or alkyl having up to 3 carbon atoms or represents a trialkylsilyl or dialkyldiphenylsilyl group, where the alkyl groups can each contain up to 4 carbon atoms.

8. Methyl 7-(4-(4-phenoxy-butyloxy)phenyl)-4-trimethylsilyloxy-hept-5-inoate.

9. 5-(3-(4-(4-Phenoxybutyloxy)-phenyl)-prop-1-in-1-yl)-tetrahydrofuran-2-one.

10. Methyl 7-(4-(4-phenoxy-butyloxy)phenyl)-4-(tetrahydropyran-2-yloxy)-5-heptinoate.

## Revendications

1. Procédé de préparation des 3-aryl-propynes de formule générale I 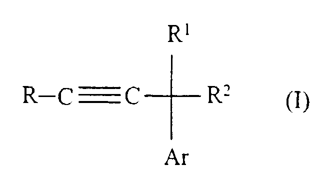 dans laquelle
Ar représente un groupe aryle en C₆-C₁₀ éventuellement substitué par des groupes alkyle, alkylsulfonyle ou alcoxycarbonyle contenant jusqu'à 10 atomes de carbone ou par des groupes alcoxy contenant jusqu'à 8 atomes de carbone, ces derniers pouvant eux-mêmes être substitués par des groupes phénoxy ou alcoxy contenant jusqu'à 6 atomes de carbone, ou bien
un noyau hétérocyclique de 5 ou 6 chaînons éventuellement condensé avec un noyau benzénique, qui peut contenir en tant qu'hétéroatomes jusqu'à 2 atomes d'azote, d'oxygène et/ou de soufre et qui peut le cas échéant être substitué par des groupes alkyle, alcoxy ou alcoxycarbonyle contenant jusqu'à 6 atomes de carbone ou par des groupes phényle,
R représente l'hydrogène ou un groupe alkyle contenant jusqu'à 10 atomes de carbone, lequel peut le cas échéant être substitué par un groupe -OR³, -NHR³ ou -NR³₂ et/ou un groupe alcoxycarbonyle contenant jusqu'à 6 atomes de carbone, ou bien
un groupe aryle en C₆-C₁₀ éventuellement substitué par des groupes alkyle, alcoxy ou alcoxycarbonyle contenant jusqu'à 8 atomes de carbone, ou bien
un groupe trialkylsilyle ou alkyldiphénylsilyle, dont les parties alkyle peuvent contenir jusqu'à 6 atomes de carbone, ou bien
un groupe de formule 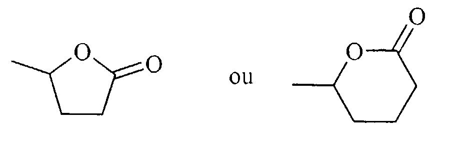
R¹, R², ayant des significations identiques ou différentes,
représentent chacun l'hydrogène, un groupe alkyle contenant jusqu'à 6 atomes de carbone ou aryle en C₆-C₁₀, ou bien
forment ensemble et avec l'atome de carbone situé entre eux, un noyau cyclopentyle ou cyclohexyle
et
R³ représente l'hydrogène, un groupe alkyle contenant jusqu'à 6 atomes de carbone, acyle contenant jusqu'à 6 atomes de carbone, benzoyle, tétrahydropyrannyle, trialkylsilyle ou alkyldiphénylsilyle dont les parties alkyle peuvent contenir chacune jusqu'à 6 atomes de carbone,
caractérisé en ce que
on fait réagir des dérivés benzyliques de formule générale II 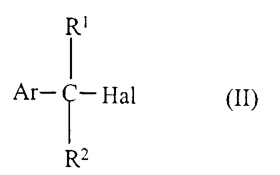 dans laquelle
Ar, R¹ et R² ont les significations indiquées en référence à la formule I, et
Hal représente le chlore ou le brome,
avec des alcynes de formule générale III
R―C≡C―R (III)
dans laquelle R a les significations indiquées en référence à la formule I,
en présence d'un dérivé du palladium, le cas échéant en présence de phosphines, à l'aide de bases faibles, dans des solvants inertes.

2. Procédé selon la revendication 1, caractérisé en ce que l'on utilise des dérivés du palladium au stade d'oxydation 0 ou +2.

3. Procédé selon les revendications 1 à 2, caractérisé en ce que l'on utilise des halogénures de palladium, des acétates de palladium ou des complexes de palladium.

4. Procédé selon les revendications 1 à 3, caractérisé en ce que l'on met en oeuvre le dérivé du palladium en quantité de 0,0001 à 0,2 éq. par rapport aux composés de formule II.

5. Procédé selon les revendications 1 à 4, caractérisé en ce que les bases utilisées sont des bases faibles non nucléophiles.

6. Procédé selon les revendications 1 à 5, caractérisé en ce que l'on prépare des 3-aryl-propynes de formule générale I dans laquelle
Ar représente un groupe phényle ou naphtyle éventuellement substitué par des groupes alkyle ou alcoxycarbonyle contenant jusqu'à 8 atomes de carbone ou par des groupes alcoxy contenant jusqu'à 6 atomes de carbone, ces derniers pouvant eux-mêmes être substitués par des groupes phénoxy ou alcoxy contenant jusqu'à 4 atomes de carbone, ou bien
un groupe thiényle, furyle, pyridyle, pyrimidyle, pyrazinyle, quinoléyle, isoquinoléyle, benzofuryle, imidazolyle, benzimidazolyle, ou indolyle qui peut le cas échéant être substitués par des groupes alkyle ou alcoxy contenant jusqu'à 4 atomes de carbone ou par des groupes phényle,
R représente l'hydrogène ou un groupe alkyle contenant jusqu'à 8 atomes de carbone, qui peut le cas échéant être substitué par un groupe -OR³, et/ou par un groupe alcoxycarbonyle contenant jusqu'à 4 atomes de carbone, ou bien
un groupe phényle ou naphtyle éventuellement substitué par des groupes alkyle ou alcoxy contenant jusqu'à 6 atomes de carbone, ou bien
un groupe trialkylsilyle ou alkyldiphénylsilyle dont les parties alkyle peuvent contenir jusqu'à 4 atomes de carbone, ou bien
un groupe de formule 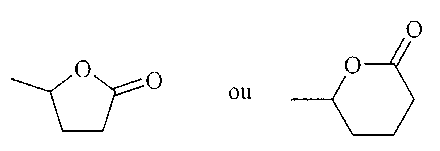
R¹, R², ayant des significations identiques ou différentes,
représentent chacun l'hydrogène, un groupe alkyle contenant jusqu'à 4 atomes de carbone ou un groupe phényle et
R³ représente l'hydrogène, un groupe alkyle contenant jusqu'à 4 atomes de carbone, acétyle ou benzoyle ou un groupe tétrahydropyrannyle, trialkylsilyle ou alkyldiphénylsilyle dont les parties alkyle peuvent contenir chacune jusqu'à 4 atomes de carbone.

7. Procédé selon les revendications 1 à 6, caractérisé en ce que l'on prépare des 3-aryl-propynes de formule générale I dans laquelle
Ar représente un groupe phényle éventuellement substitué par des groupes alkyle contenant jusqu'à 4 atomes de carbone ou alcoxy contenant jusqu'à 6 atomes de carbone, ces derniers pouvant eux-mêmes être substitués par des groupes phénoxy ou alcoxy contenant jusqu'à 3 atomes de carbone,
R représente l'hydrogène ou un groupe alkyle contenant jusqu'à 6 atomes de carbone, éventuellement substitué par un groupe -OR³ et/ou par un groupe alcoxycarbonyle contenant jusqu'à 4 atomes de carbone, ou bien un groupe phényle éventuellement substitué par des groupes alkyle ou alcoxy contenant jusqu'à 4 atomes de carbone, ou bien un groupe de formule 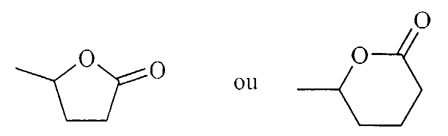
ou bien un groupe triméthylsilyle, tert-butylsilyle ou diphénylméthylsilyle,
R¹, R², représentent l'hydrogène ou des groupes méthyle et
R³ représente l'hydrogène ou un groupe alkyle contenant jusqu'à 3 atomes de carbone ou un groupe trialkylsilyle ou dialkyldiphènylsilyle dont les parties alkyle peuvent contenir chacune jusqu'à 4 atomes de carbone.

8. Le 7-(4-(4-phénoxy-butyloxy)-phényl)-4-triméthylsilyloxy-hept-5-ynoate de méthyle.

9. La 5-(3-(4-(4-phénoxy-butyloxy)-phényl-prop-1-yn-1-yl)-tétrahydrofuranne-2-one.

10. Le 7-(4-(4-phénoxy-butyloxy)-phényl)-4-(tétrahydropyranne-2-yloxy)-hept-5-ynoate de méthyle.
